(19) **Europäisches Patentamt**
**European Patent Office**
**Office européen des brevets**

(11) **EP 3 572 126 B1**

(12) **EUROPEAN PATENT SPECIFICATION**

(45) Date of publication and mention
of the grant of the patent:
**12.06.2024 Bulletin 2024/24**

(21) Application number: **18729508.4**

(22) Date of filing: **16.01.2018**

(51) International Patent Classification (IPC):
**A61Q 5/08** *(2006.01)* **A61K 8/81** *(2006.01)*
**A61K 8/06** *(2006.01)* **A61K 8/92** *(2006.01)*

(52) Cooperative Patent Classification (CPC):
**A61Q 5/08; A61K 8/062; A61K 8/8111;**
**A61K 8/8164; A61K 8/92**

(86) International application number:
**PCT/RU2018/000008**

(87) International publication number:
**WO 2018/135974 (26.07.2018 Gazette 2018/30)**

(54) **FORMULATION FOR BLEACHING HAIR**

FORMULIERUNG ZUM BLEICHEN VON HAAREN

FORMULATION POUR LA DÉCOLORATION DES CHEVEUX

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **23.01.2017 RU 2017103432**

(43) Date of publication of application:
**27.11.2019 Bulletin 2019/48**

(73) Proprietor: **Obshchestvo S Ogranichennoj**
**Otvetstvennostyu "Yunikosmetik"**
**St.Petersburg 195273 (RU)**

(72) Inventor: **CHISTYAKOVA, Olga Ivanovna**
**St.Petersburg 193312 (RU)**

(74) Representative: **Viering, Jentschura & Partner**
**mbB**
**Patent- und Rechtsanwälte**
**Am Brauhaus 8**
**01099 Dresden (DE)**

(56) References cited:
WO-A1-2009/010356      WO-A1-2015/028007
WO-A1-2017/129234      WO-A2-2008/156723
WO-A2-2009/134875      US-A1- 2011 236 331

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

TECHNICAL FIELD

The invention relates to cosmetic industry, namely to production of hair bleaching formulations. The proposed composition which is intended for use in multi-phase hair bleaching formulation consisting of separate phases to be mixed directly before use and which contains the mixture of polyisobutene and the mixture of hydrogenated polyisobutene with ethylene/propylene/styrene copolymer and butylene/ethylene/styrene copolymer. The proposed invention relates to hair bleaching formulation which consists of three separately-located phases to be mixed directly before use and contains the specified composition the mixture of polyisobutene and the mixture of hydrogenated polyisobutene with ethylene/propylene/styrene copolymer and butylene/ethylene/styrene copolymer as a phase.

PRIOR ART

Hair bleaching is destruction of natural melanin pigment or cosmetic pigment which determine the color of hair. It can be done by two methods: a reduction process and an oxidation process.
The reduction method mainly employs sulfur-containing reducing agents such as sodium dithionite (e.g. see US 2017/128334 A1); derivatives of sulfinic acid (*e.g.* see DE 10 2006 053 343 A1) or sulfonic acid (e.g. see DE 10 2006 022 214 A1); organic compounds with thiol groups and substituted carbonyl group such as cysteine (e.g. see EP 1 430 873 A1); etc.
But the most widely used method is an oxidation process. It insures more efficient bleaching and nicely combines with subsequent hair coloring with oxidation dyes. The proposed oxidizers are enzymes like oxidoreductase (e.g. see US 6,273,920 B1); ethylenediamine-N,N-disuccinic acid and its succinates (e.g. see FR 2 870 736 B1); mixture of alkali salts of peroxy acid and magnesium carbonate such as the mixture of sodium and/or potassium persulphate with magnesium carbonate (e.g. see GB 1,059,986 A); peroxygenated salts (e.g. see WO 2011/121010 A1); sodium and/or potassium peroxydiphosphates (e.g. see US 3,649,159 A); carboxylic ethers mixed with persulphates or peroxydisulfates (e.g. see DE 10 2004 030 178 A1); mixture of ammonimum peroxydisulfate with specially prepared watersoluble glass (e.g. see DE 10 2005 019 420 A1); and others.
The most widely used substance for human hair bleaching is hydrogen peroxide. General wide use of hydrogen peroxide for this purpose is caused by the following reasons:

- it bleaches pigments in human hair within a relatively short time;
- it can be used safely if proper precautions are observed;
- a bleaching process can be easily controlled;
- no undesirable by-products are produced.

Hydrogen peroxide is active in an alkaline environment (pH 8.1-10.7). The most preferable alkaline is usually ammonia-water mixture though alkylamines can be used too.
Hydrogen peroxide, like most of peroxides, is easily decomposed when contacting with heavy metals, such as iron, copper, manganese, and can quickly become ineffective. This drawback is usually eliminated by introduction of some chelant into the formulation, e.g. ethylenediaminetetraacetic acid (EDTA). Bleaching is accompanied by emerging of reddish shades on hair. In order to reduce or neutralize a "copper" shade of bleached hair, blue or violet colorants are introduced into formulations.
The most consistent and controlled results can be achieved if a formulation has a form of viscous fluid, emulsion or paste. In recent years, manufacturers have tend to create formulations consisting of two components such as aqueous solution or emulsion containing hydrogen peroxide, and a hard powder containing persulfate salts which amplify the bleaching effect of peroxide compounds. In order to prevent dustiness of such powders, a certain amount of mineral oil can be introduced in them (e.g. see US 5,989,530 A).
Frequent bleaching of hair can make it dry and fragile, and breaking strength significantly decreases. That is why bleaching formulations are enriched with conditioning additives which improve appearance of hair but still do not eliminate all the problems mentioned above.
US 2007/0169285 A1 discloses a formulation for hair bleaching that consists of two phases located separately from each other and mixed directly before use. Further hair bleaching formulations comprising two phases are disclosed in WO 2009/134875 A2. The authors propose adding the third oil phase (also located in an individual container) to the two-phase formulation which consists of powder containing persulfates of alkaline metals and ammonium, alkaline agent sodium metasilicate and other usual components for bleaching formulations and of hydrogen peroxide aqueous solution (these components being located in different containers). The authors suppose that oil would cover hair and penetrate into it by filling submicroscopic pores which significantly increase during bleaching due to the effect of alkaline agents destroying disulfide bonds of keratin. Thus, oil would perform protective function and prevents hair breakage.

But the authors of this invention discovered that oil, which is not substantive to hair keratin, does not create a uniform protective coating on hair surface and is partly removed from it by abrasive particles of powder and by tools used during bleaching (a brush, a comb), which calls for improvement of the solution disclosed in prior art.

The claimed invention is aimed at overcoming the problems of the prior art. The authors of this invention discovered that damage of hair during bleaching can be reduced by introduction of polyisobutene mixed with hydrogenated polyisobutene, ethylene/propylene/styrene copolymer and butylene/ethylene/styrene copolymer into the oil phase of bleaching formulation.

SUMMARY

The invention discloses a A hair bleaching formulation which consists of three phases located separately from each other and mixed directly before use, where:

- Phase A is a powder containing potassium persulfate, ammonium persulfate and sodium metasilicate;
- Phase B is aqueous solution containing hydrogen peroxide in an acidic environment;
- Phase C is oil phase containing mineral oil, mixture of polyisobutene with mixture of hydrogenated polyisobutene with ethylene/propylene/styrene copolymer and butylene/ethylene/styrene copolymer, and conditioning agent is phenyl trimethicone, preservative agent is phenoxyethanol.

A hair bleaching formulation may further be characterized in that its components in Phase C are in the following ratios, % by weight:

| | |
|---|---|
| polyisobutene | 10.0 - 35.0; |
| mixture of hydrogenated polyisobutene with ethylene/propylene/styrene copolymer and butylene/ethylene/styrene copolymer | 10.0 - 35.0; |
| mineral oil | up to 100. |

A hair bleaching formulation may further be characterized in that the content of phenyl trimethicone in Phase C is 1.0-15.0% by weight.

A hair bleaching formulation may further be characterized in that the content of phenoxyethanol in Phase C is 0.1-1.0% by weight.

A hair bleaching formulation may further be characterized in that the components in Phase B are in the following ratios, % by weight:

| | |
|---|---|
| hydrogen peroxide | 1.0 - 12.0; |
| cetearyl alcohol | 0.5 - 10.0; |
| ceteareth-20 | 0.1 - 8.0; |
| cetrimonium chloride | 0.1 - 5.0; |
| sodium stannate | 0.01 - 1.0; |
| phosphoric acid | 0.01 - 1.0; |
| water | up to 100. |

A hair bleaching formulation may further be characterized in that the components in Phase A are in the following ratios, % by weight:

| | |
|---|---|
| sodium metasilicate | 5.0 - 30.0; |
| ammonium persulfate | 10.0 - 40.0; |
| potassium persulfate | up to 100. |

A hair bleaching formulation may further be characterized in that Phase A additionally contains inorganic fillers, thickeners, surfactants, mineral oil, complexones, perfume compositions and/or colorants, and pigments.

A hair bleaching formulation may further be characterized in that inorganic fillers in Phase A are selected from a group consisting of magnesium oxide, titanium dioxide, silica dioxide.

A hair bleaching formulation may further be characterized in that the thickener in Phase A is selected from a group consisting of xanthan gum, guar hydroxypropyltrimonium chloride.

A hair bleaching formulation may further be characterized in that the content of guar hydroxypropyltrimonium chloride in Phase A is 0.1-5.0% by weight.

A hair bleaching formulation may further be characterized in that the surfactant in Phase A is selected from sodium lauryl sulfate.

A hair bleaching formulation may further be characterized in that the content of sodium lauryl sulfate in Phase A is 0.1-5.0% by weight.

A hair bleaching formulation may further be characterized in that the complexone in Phase A is selected from ethylenediaminetetraacetic acid tetrasodium.

A hair bleaching formulation may further be characterized in that the content of ethylenediaminetetraacetic acid tetrasodium in Phase A is 0.1-1.0% by weight.

A hair bleaching formulation may further be characterized in that the colorant in Phase A is selected from ultramarine.

A hair bleaching formulation may further be characterized in that the content of ultramarine in Phase A is 0.1-1.0% by weight.

A hair bleaching formulation may further be characterized in that:

- Phase A contains, % by weight:

| | |
|---|---|
| sodium metasilicate | 5.0 - 30.0; |
| ammonium persulfate | 10.0 - 40.0; |
| potassium persulfate | up to 100; |

- Phase B contains, % by weight:

| | |
|---|---|
| hydrogen peroxide | 1.0 - 12.0; |
| water | up to 100; |

- Phase C contains, % by weight:

| | |
|---|---|
| polyisobutene | 10.0 - 35.0; |
| mixture of hydrogenated polyisobutene with ethylene/propylene/styrene copolymer and butylene/ ethylene/styrene copolymer | 10.0 - 35.0; |
| mineral oil | up to 100. |

A hair bleaching formulation characterized in that it is intended for bleaching of light hair and that Phase A, Phase B and Phase C are in the ratios of 6:12:1.

A hair bleaching formulation characterized in that it consists of three phases located separately from each other and each phase - Phase A, Phase B and Phase C - is placed in an individual container.

DETAILED DESCRIPTION

This invention is aimed at addressing the problem of hair damage during bleaching. The reduction of hair damage during bleaching is achieved by introduction of mixture of polyisobutene with mixture of hydrogenated polyisobutene, ethylene/propylene/styrene copolymer and butylene/ethylene/styrene copolymer into the oil phase of the hair bleaching product.

Polyisobutene is a cosmetically acceptable, commercially-available product (e.g. REWOPAL® PIB 1000 from Evonik Industries AG Personal Care) and represents a thermoplastic polymer in the form of transparent fluid which is not soluble in water but soluble in oil. It has softening and moisturizing effect, and prevents moisture loss. It is usually used as a film-forming agent, moisturizer, thickener, softening and/or binding component.

Hydrogenated polyisobutene is a cosmetically acceptable, commercially-available product (e.g. LUVITOL® LITE from BASF) and represents colorless transparent fluid. It gives silky feel on skin and is used as an alternative for mineral oil. Non-comedogenic emollient with moisturizing and moisture repellency characteristics, not irritating eyes or skin.

Hydrogenated polyisobutene mixed with ethylene/propylene/styrene copolymer and butylene/ethylene/styrene copolymer is a cosmetically acceptable, commercially-available product, e.g. under trademark Versagel® ME: Hydrogenated Polyisobutene (and) Ethylene/Propylene/Styrene Copolymer (and) Butylene/Ethylene/Styrene Copolymer from Calumet Penreco Personal Care Products (Karns City, PA 16041, USA). Various series of Versagel® Me may be used, such as ME 500, ME 750, ME 16000, ME 2000, which differ in viscosity depending on the required quantity and quality of components of the claimed invention.

The authors of this invention discovered that polyisobutene dissolved in mineral oil, the adhesion properties of which are modified by hydrogenated polyisobutene mixed with ethylene/propylene/styrene copolymer and butylene/ethylene/styrene copolymer to the desired level, forms a uniform hydrophobic film with high mechanical strength on the surface of hair. Such a film prevents contact of hair with solution of alkaline agent which is used for decomposition of hydrogen peroxide and peroxide salts and seriously damages hair. At the same time the film permits free penetration of gaseous products of decomposition of peroxide compounds: molecular and atomic oxygen and ammonia which are required for hair bleaching. Thanks to its high adhesion properties, the film remains on hair for a long time and provides the necessary protection for hair during bleaching.

The invention relates to the hair bleaching formulation which consists of three phases separate from each other and mixed directly before use The invention makes it possible to reduce hair damage during bleaching and to preserve hair strength, suppleness and a healthy appearance.

The hair bleaching formulation of the invention consists of three phases located separately from each other and mixed directly before use (i.e. the formulation of the invention is principally a set, in which every phase is placed in a separated package (container), e.g. a bottle, or the formulation of the invention is principally a combination of phases, when hair can be bleached by mixing of the phases directly before use), where:

- Phase A is a powder containing potassium persulfate, ammonium persulfate and sodium metasilicate;
- Phase B is aqueous solution containing hydrogen peroxide in acidic environment;
- Phase C is principally the above composition of the invention that comprises oil phase containing mineral oil, polyisobutene, mixture of hydrogenated polyisobutene with ethylene/propylene/styrene copolymer and butylene/ethylene/styrene copolymer.

In the hair bleaching formulation of the invention the components in Phase C can be contained in the following ratios, % by weight:

| | |
|---|---|
| polyisobutene | 10.0 - 35.0; |
| mixture of hydrogenated polyisobutene with ethylene/propylene/styrene copolymer and butylene/ethylene/styrene copolymer | 10.0 - 35.0; |
| mineral oil | up to 100. |

Preferably, the content of polyisobutene can vary from 10.0 to 35.0% by weight, more preferably - 15.0-30.0% by weight, even more preferably - 20.0-25.0% by weight, the most preferably - 22.5% by weight.

Preferably, the content of the mixture of hydrogenated polyisobutene with ethylene/propylene/styrene copolymer and butylene/ethylene/styrene copolymer can vary from 10.0 to 35.0% by weight, more preferably - 15.0-30.0% by weight, even more preferably - 20.0-25.0% by weight, the most preferably - 22.5% by weight.

In the hair bleaching formulation of the invention Phase C can additionally contain a conditioning agent. The conditioning agent in Phase C can constitute phenyl trimethicone. The content of the specified phenyl trimethicone in Phase C can vary within 1.0-15.0 by weight, preferably from 1.0 to 15.0% by weight, more preferably - 5.0-13.0% by weight, even more preferably - 9.0-11.0% by weight, the most preferably - 10.0% by weight. Alternatively, the conditioning agent can constitute amodimethicone, dimethicone or any acceptable and traditionally used conditioning agents.

In the hair bleaching formulation of the invention Phase C can additionally contain a preservative agent. The preservative agent in Phase C can constitute phenoxyethanol. The content of the specified phenoxyethanol in Phase C can vary within 0.1-1.0 by weight, preferably from 0.1 to 1.0% by weight, more preferably - 0.2-0.8% by weight, even more preferably - 0.4-0.6% by weight, the most preferably - 0.5% by weight. Alternatively, the preservative agent can constitute methylparaben, propylparaben or any acceptable and traditionally used preservative agents.

Phase B is aqueous solution containing oxidizing agent selected from hydrogen peroxide solution in an acidic environment. Moreover, percarbonates or sodium/potassium/ammonium perborates, barium peroxide, urea hydrogen peroxide (complex of hydrogen peroxide with urea) can be used as an oxidizing agent.

In the hair bleaching formulation of the invention the content of hydrogen peroxide in Phase B can vary within 1.0-12.0% by weight. Preferably, the content of hydrogen peroxide is from 1.0 to 12.0% by weight, more preferably - 3.0-9.0% by weight, even more preferably - 5.0-7.0% by weight, the most preferably - 6.0% by weight.

In the hair bleaching formulation of the invention Phase B can additionally contain cetearyl alcohol, ceteareth-20, cetrimonium chloride, sodium stannate and phosphoric acid. Phase B can additionally contain, as emulsifiers, fatty alcohols selected e.g. from cetyl alcohol, stearyl alcohol, cetearyl alcohol and others, fatty esters and acids, non-ionic surfactants selected e.g. from ceteareth-6, ceteareth-20, ceteareth-30, ceteareth-25, trideceth-6, trideceth-9, trideceth-12 and others. Cationic surfactant selected e.g. from cetrimonium chloride, behentrimonium chloride and others.

In the hair bleaching formulation of the invention the content of cetearyl alcohol in Phase B can very within 0.5-10.0% by weight. Preferably, the content of cetearyl alcohol is from 0.5 to 10.0% by weight, more preferably - 1.0-9.0% by weight, even more preferably - 3.0-8.0% by weight, the most preferably - 5.5% by weight.

In the hair bleaching formulation of the invention the content of ceteareth-20 in Phase B can very within 0.1 -8.0% by weight. Preferably, the content of ceteareth-20 is from 0.1 to 8.0% by weight, more preferably - 0.2-5.0% by weight, even more preferably - 0.3-2.0% by weight, the most preferably - 0.9% by weight.

In the hair bleaching formulation of the invention the content of sodium stannate in Phase B can vary within 0.01-1.0% by weight. Preferably, the content of sodium stannate is from 0.01 to 1.0% by weight, more preferably - 0.1-0.9% by weight, even more preferably - 0.2-0.6% by weight, the most preferably - 0.4% by weight.

In the hair bleaching formulation of the invention the content of phosphoric acid in Phase B can very within 0.01-1.0% by weight. Preferably, the content of phosphoric acid is from 0.01 to 1.0% by weight, more preferably - 0.1-0.9% by weight, even more preferably - 0.2-0.7% by weight, the most preferably - 0.4% by weight.

Preferably, in the hair bleaching formulation of the invention the components in Phase B can be contained in the following ratios, % by weight:

| | |
|---|---|
| hydrogen peroxide | 1.0 - 12.0; |
| cetearyl alcohol | 0.5 - 10.0; |
| ceteareth-20 | 0.1 - 8.0; |
| sodium stannate | 0.01 - 1.0; |
| phosphoric acid (concentrated) | 0.01 - 1.0; |
| water | up to 100. |

Phase A is a powder containing oxidizers, e.g. potassium persulfate, ammonium persulfate and their mixtures, and alkaline agent selected from sodium metasilicate. Moreover, percarbonates or sodium/potassium/ammonium perborates, barium peroxide can be used as oxidizers (oxidizing agents). Moreover, sodium and potassium hydroxide, as well as any solid alkaline agents can be used as alkaline agents.

In the hair bleaching formulation of the invention the components in Phase A can be contained in the following ratios, % by weight:

| | |
|---|---|
| sodium metasilicate | 5.0 - 30.0; |
| ammonium persulfate | 10.0 - 40.0; |
| potassium persulfate | up to 100. |

Preferably, the content of sodium metasilicate is from 5.0 to 30.0% by weight, more preferably - 7.0-20.0% by weight, even more preferably - 11.0-16.0% by weight, the most preferably - 13.5% by weight.

Preferably, the content of ammonium persulfate is from 10.0 to 40.0% by weight, more preferably - 11.0-30.0% by weight, even more preferably - 12.0-20.0% by weight, the most preferably - 16.0% by weight.

Phase A can be made either as powder or heterogeneous cream, paste, etc., containing this powder.

Phase A can contain other components usually used in bleaching compounds, as well as mineral oil to prevent dust formation.

In the hair bleaching formulation of the invention Phase A can additionally contain inorganic fillers, thickeners, surfactants, mineral oils, complexones, perfume compositions and/or colorants, pigments.

Inorganic fillers in Phase A of the hair bleaching formulation of the invention can be selected from a group consisting of magnesium oxide, titanium dioxide, silica dioxide (also known as silicon dioxide or silicon oxide). Alternatively, the inorganic fillers can constitute magnesium carbonate, talc or any acceptable and traditionally used inorganic fillers.

In the hair bleaching formulation of the invention the content of magnesium oxide in Phase A can vary within 0.1-10.0% by weight. Preferably, the content of magnesium oxide is from 0.1 to 10.0% by weight, more preferably - 0.8-7.0% by weight, even more preferably - 0.9-4.0% by weight, the most preferably - 2.5% by weight.

In the hair bleaching formulation of the invention the content of titanium dioxide in Phase A can vary within 0.1-10.0% by weight. Preferably, the content of titanium dioxide is from 0.1 to 10.0% by weight, more preferably - 0.8-7.0% by weight, even more preferably - 0.9-4.0% by weight, the most preferably - 2.5% by weight.

In the hair bleaching formulation of the invention the content of silica dioxide in Phase A can vary within 0.1-10.0% by weight. Preferably, the content of silica dioxide is from 0.1 to 10.0% by weight, more preferably - 0.8-7.0% by weight, even more preferably - 0.9-4.0% by weight, the most preferably - 2.5% by weight.

In the hair bleaching formulation of the invention the thickeners in Phase A can be selected from a group consisting of xanthan gum, guar hydroxypropyltrimonium chloride. Moreover, the thickeners can be selected from hydroxyethyl cellulose, starches, gellan gum, carageenan.

In the hair bleaching formulation of the invention the content of xanthan gum in Phase A can vary within 0.1-5.0% by weight. Preferably, the content of xanthan gum is from 0.1 to 5.0% by weight, more preferably - 0.8-4.0% by weight, even more preferably - 0.9-3.0% by weight, the most preferably - 2.0% by weight.

In the hair bleaching formulation of the invention the content of guar hydroxypropyltrimonium chloride in Phase A can vary within 0.1-5.0% by weight. Preferably, the content of guar hydroxypropyltrimonium chloride is from 0.1 to 5.0% by weight, more preferably - 0.8-4.0% by weight, even more preferably - 0.9-3.0% by weight, the most preferably - 2.0% by weight.

In the hair bleaching formulation of the invention the surfactant in Phase A can be selected from sodium lauryl sulfate. Alternatively, the surfactant can constitute sodium lauroyl sarcosinate (sodium lauryl sarcosinate), disodium lauryl sulfosuccinate, sodium cocoyl isethionate or any acceptable and traditionally used surfactants.

In the hair bleaching formulation of the invention the content of sodium lauryl sulfate in Phase A can vary within 0.1-5.0% by weight. Preferably, the content of sodium lauryl sulfate is from 0.1 to 5.0% by weight, more preferably - 0.8-4.0% by weight, even more preferably - 0.9-3.0% by weight, the most preferably - 2.0% by weight.

In the hair bleaching formulation of the invention the complexone in Phase A can be selected from ethylenediaminetetraacetic acid tetrasodium. Alternatively, the complexone can constitute L-glutamic acid N,N-diacetic acid tetra sodium salt, nitrilotriacetic acid sodium salt or any acceptable and traditionally used complexones.

In the hair bleaching formulation of the invention the content of ethylenediaminetetraacetic acid tetrasodium in Phase A can vary within 0.1-1.0% by weight. Preferably, the content of ethylenediaminetetraacetic acid tetrasodium is from 0.1 to 1.0% by weight, more preferably - 0.2-0.8% by weight, even more preferably - 0.4-0.6% by weight, the most preferably - 0.5% by weight.

In the hair bleaching formulation of the invention the colorant in Phase A can be selected from ultramarine.

In the hair bleaching formulation of the invention the content of ultramarine in Phase A can vary within 0.1-1.0% by weight. Preferably, the content of ultramarine is from 0.1 to 1.0% by weight, more preferably - 0.2-0.8% by weight, even more preferably - 0.4-0.6% by weight, the most preferably - 0.5% by weight.

Mineral oil in the hair bleaching formulation of the invention can constitute commercially-available mineral oil (e.g. POWEROIL White Oil Pharma 15 from Apar Industries Limited, Savonol 20 from Savita Chemicals). In the hair bleaching formulation of the invention the content of mineral oil in Phase A can vary within 0.1-10.0% by weight. Preferably, the content of mineral oil is from 0.1 to 10.0% by weight, more preferably - 1.0-9.0% by weight, even more preferably - 3.0-8.0% by weight, the most preferably - 5.5% by weight.

In the hair bleaching formulation of the invention the content of perfume composition in Phase A can vary within 0.1-1.5% by weight. Preferably, the content of perfume composition is from 0.1 to 1.5% by weight, more preferably - 0.5-1.1% by weight, even more preferably - 0.7-0.9% by weight, the most preferably - 0.8% by weight.

In the hair bleaching formulation of the invention, the components can be contained in the following ratios, % by weight:

- Phase A contains, % by weight:

| | |
|---|---|
| sodium metasilicate | 5.0-30.0; |
| ammonium persulfate | 10.0-40.0; |
| potassium persulfate | up to 100; |

- Phase B contains, % by weight:

| | |
|---|---|
| hydrogen peroxide | 1.0-12.0; |
| water | up to 100; |

- Phase C contains, % by weight:

| | |
|---|---|
| polyisobutene | 10.0-35.0; |
| mixture of hydrogenated polyisobutene with ethylene/propylene/styrene copolymer and butylene/ethylene/styrene copolymer | 10.0-35.0; |
| mineral oil | up to 100. |

If the hair bleaching formulation of the invention is intended for bleaching of light hair, the authors discovered that the best results are achieved, when Phase A, Phase B and Phase C are in the ratio of exactly of approximately 6:12:1.

If the hair bleaching formulation of the invention is intended for bleaching of dark hair, the authors discovered that the best results are achieved, when Phase A, Phase B and Phase C are in the ratio of exactly of approximately 6:18:1.

The hair bleaching formulation of the invention consists of three phases located separately from each other and each phase - Phase A, Phase B and Phase C - can be placed in an individual container.

EMBODIMENTS OF THE INVENTION

The claimed invention can be illustrated by the following non-limiting examples.

Example 1.

This example in Table 1 illustrates formulation No 1 of Phase A within the scope of the claimed invention.

Table 1. Formulation of Phase A.

| Ingredient | Quantity, % by weight |
|---|---|
| sodium metasilicate | 5.0 |
| ammonium persulfate | 10.0 |
| potassium persulfate | up to 100 |
| magnesium oxide | 0.1 |
| titanium dioxide | 0.1 |
| silica dioxide | 0.1 |
| xanthan gum | 0.1 |
| guar hydroxypropyltrimonium chloride | 0.1 |
| sodium lauryl sulfate | 0.1 |
| mineral oil | 4.0 |
| ethylenediaminetetraacetic acid tetrasodium | 0.01 |
| perfume composition | 0.1 |
| ultramarine | 0.1 |

Example 2.

This example in Table 2 illustrates formulation No 2 of Phase A within the scope of the claimed invention.

Table 2. Formulation of Phase A.

| Ingredient | Quantity, % by weight |
|---|---|
| sodium metasilicate | 30.0 |
| ammonium persulfate | 40.0 |
| potassium persulfate | up to 100 |
| magnesium oxide | 1.0 |
| titanium dioxide | 1.0 |
| silica dioxide | 1.0 |
| xanthan gum | 1.0 |
| guar hydroxypropyltrimonium chloride | 1.0 |
| sodium lauryl sulfate | 1.0 |
| mineral oil | 5.0 |
| ethylenediaminetetraacetic acid tetrasodium | 0.5 |
| perfume composition | 0.5 |
| ultramarine | 0.5 |

Example 3.

This example in Table 3 illustrates formulation No 1 of Phase B within the scope of the claimed invention.

Table 3. Formulation of Phase B.

| Ingredient | Quantity, % by weight |
|---|---|
| hydrogen peroxide | 1.0 |
| cetearyl alcohol | 2.5 |
| ceteareth-20 | 0.1 |
| sodium stannate | 0.1 |
| phosphoric acid | 0.1 |
| water | up to 100 |

Example 4.

This example in Table 4 illustrates formulation No 2 of Phase B within the scope of the claimed invention.

Table 4. Formulation of Phase B.

| Ingredient | Quantity, % by weight |
|---|---|
| hydrogen peroxide | 12.0 |
| cetearyl alcohol | 9.0 |
| ceteareth-20 | 8.0 |
| sodium stannate | 1.0 |
| phosphoric acid | 0.5 |
| water | up to 100 |

Example 5.

This example in Table 5 illustrates formulation No 1 of Phase C within the scope of the claimed invention. Polyisobutene and mixture of hydrogenated polyisobutene with ethylene/propylene/styrene copolymer and butylene/ethylene/styrene copolymer are in the ratio of 1:1.

Table 5. Formulation of Phase C.

| Ingredient | Quantity, % by weight |
|---|---|
| polyisobutene | 10.0 |
| mixture of hydrogenated polyisobutene with ethylene/propylene/styrene copolymer and butylene/ethylene/styrene copolymer | 10.0 |
| phenyl trimethicone | 1.0 |
| phenoxyethanol | 0.1 |
| mineral oil | up to 100 |

Example 6.

This example in Table 6 illustrates formulation No 2 of Phase C within the scope of the claimed invention. Polyisobutene and mixture of hydrogenated polyisobutene with ethylene/propylene/styrene copolymer and butylene/ethylene/styrene copolymer are in the ratio of 1:1.

Table 6. Formulation of Phase C.

| Ingredient | Quantity, % by weight |
|---|---|
| polyisobutene | 35.0 |

(continued)

| Ingredient | Quantity, % by weight |
|---|---|
| mixture of hydrogenated polyisobutene with ethylene/propylene/styrene copolymer and butylene/ethylene/styrene copolymer | 35.0 |
| phenyl trimethicone | 15.0 |
| phenoxyethanol | 1.0 |
| mineral oil | up to 100 |

Example 7.

This example in Table 7 illustrates formulation No 3 of Phase C within the scope of the claimed invention. Polyisobutene and mixture of hydrogenated polyisobutene with ethylene/propylene/styrene copolymer and butylene/ethylene/styrene copolymer are not in the ratio of 1:1.

Table 7. Formulation of Phase C.

| Ingredient | Quantity, % by weight |
|---|---|
| polyisobutene | 10.0 |
| mixture of hydrogenated polyisobutene with ethylene/propylene/styrene copolymer and butylene/ethylene/styrene copolymer | 35.0 |
| phenyl trimethicone | 1.0 |
| phenoxyethanol | 0.1 |
| mineral oil | up to 100 |

Example 8.

This example in Table 8 illustrates formulation No 4 of Phase C within the scope of the claimed invention. Polyisobutene and mixture of hydrogenated polyisobutene with ethylene/propylene/styrene copolymer and butylene/ethylene/styrene copolymer are not in the ratio of 1:1.

Table 8. Formulation of Phase C.

| Ingredient | Quantity, % by weight |
|---|---|
| polyisobutene | 35.0 |
| mixture of hydrogenated polyisobutene with ethylene/propylene/styrene copolymer and butylene/ethylene/styrene copolymer | 10.0 |
| phenyl trimethicone | 1.0 |
| phenoxyethanol | 0.1 |
| mineral oil | up to 100 |

Example 9. The best embodiment of the invention

This example in Table 9 illustrates the best embodiment of the invention within the scope of the claimed invention.

Table 9. The best embodiment of the invention

| Ingredient | Quantity, % by weight |
|---|---|
| Phase A: sodium metasilicate | 10.0 |
| ammonium persulfate | 19.0 |
| potassium persulfate | 58.0 |
| magnesium oxide | 1.0 |
| titanium dioxide | 1.0 |

(continued)

| Ingredient | Quantity, % by weight |
|---|---|
| Phase A: | |
| silica dioxide | 1.0 |
| xanthan gum | 1.0 |
| guar hydroxypropyltrimonium chloride | 1.0 |
| sodium lauryl sulfate | 1.0 |
| mineral oil | 6.8 |
| ethylenediaminetetraacetic acid tetrasodium perfume composition | 0.1 |
| ultramarine | 0.1 |
| Phase B: | |
| hydrogen peroxide | 10.0 |
| cetearyl alcohol | 2.0 |
| ceteareth-20 | 1.0 |
| cetrimonium chloride | 1.0 |
| sodium stannate | 1.0 |
| phosphoric acid (concentrated) | 1.0 |
| water | 84.0 |
| Phase C: | |
| polyisobutene | 27.0 |
| mixture of hydrogenated polyisobutene with ethylene/propylene/styrene copolymer and butylene/ethylene/styrene copolymer | 27.0 |
| phenyl trimethicone | 15.0 |
| phenoxyethanol | 1.0 |
| mineral oil | 30.0 |

Example 10.

This example in Table 10 illustrates formulation No 1 for hair bleaching within the scope of the claimed invention that contains components of the phases in the ratios specified in Example 9.

Table 10. Formulation for hair bleaching.

| Phase | Quantity, parts |
|---|---|
| Phase A | 6 |
| Phase B | 12 |
| Phase C | 1 |

Example 11.

This example in Table 11 illustrates formulation No 2 for hair bleaching within the scope of the claimed invention, that contains components of the phases in the ratios specified in Example 9.

Table 11. Formulation for hair bleaching.

| Phase | Quantity, parts |
|---|---|
| Phase A | 6 |
| Phase B | 18 |
| Phase C | 1 |

Example 12. Preparation of hair bleaching formulation.

Powder (Phase A).

The calculated quantities of ammonium persulfate, potassium persulfate, sodium metasilicate, magnesium oxide, titanium dioxide, silica dioxide, ethylenediaminetetraacetic acid tetrasodium, xanthan gum, guar hydroxypropyltrimonium chloride, sodium lauryl sulfate and ultramarine are loaded to a suitable apparatus and mixed until complete homogenization.

The calculated quantity of perfume composition dissolved in mineral oil is introduced into the mass through atomizing cones with constant stirring, a sample is taken for analysis, and in the event of a positive result the product is packed in special packaging.

Oxidizing Agent (Phase B).

The calculated quantity of water is heated in the proper apparatus to the temperature of 75-80 °C, then the calculated quantities of sodium stannate, cetearyl alcohol and ceteareth-20 are added. The mixture is stirred for 30 minutes until complete dissolution, and the calculated quantity of cetrimonium chloride is added. The product is stirred for 60 minutes, cooled to a temperature of 35-40 °C, the calculated quantity of preliminarily prepared aqueous solution of phosphoric acid is introduced, and then hydrogen peroxide is added.

The mass is stirred for 30 minutes, a sample is taken for analysis, and in the event of positive result the product is packed into special packaging.

Oil Phase (Phase C).

The calculated quantities of polyisobutene and Versagel® ME are loaded into the suitable apparatus, stirred at a temperature of 65-70 °C until complete homogenization.

The calculated quantity of mineral oil is added to the resulting mixture, everything is stirred until complete dissolution and the calculated quantity of phenyl trimethicone is loaded. The mass is stirred until complete dissolution of components, cooled to a temperature of 35-40 °C and the calculated quantity of phenoxyethanol is added. The mass is stirred for 60 minutes, then a sample is taken for analysis and in the event of a positive result, the product is packed into special packaging.

Example 13.

A method of hair bleaching that includes application of the hair bleaching formulation of the invention on hair.

Directly before use Phase A is mixed with Phase B until uniform consistency is achieved, then Phase C is added. The resulting mixture is applied on hair according to the procedures traditionally used in this sphere.

Example 14.

The experiments for studying stress-related characteristics of examined materials in the mode of uniaxial tension were conducted on an Instron-1122 universal machine. This device makes it possible to vary loading rate from 0.05 mm/min to 1000 mm/min and the load range from $10^{-3}N$ to $5\times10^{3}N$. The recording instrument of the machine records the diagram of tension as a function of elongation $P(\Delta l)$ (where P - load, N; $\Delta l$ - absolute elongation of a sample, mm).

The samples were tested at the specified elongation rate of 10 mm/min The obtained data were transformed into functions $\sigma(\varepsilon)$ ($\sigma$ - tension stress, N/mm$^2$, $\varepsilon$ - relative elongation, %), where:

$$\sigma_i = F/P,$$

where F - cross-section area of human hair that is determined as:

$$F = \pi \cdot D_i^2/4, \ mm^2,$$

where $D_i$ - diameterof hair, mm.

$$\varepsilon_i = \Delta l / l_0 \cdot 100\%,$$

where $l_0$ - initial length of a sample, equal to 30 mm.

An averaged load-elongation curve for a single human hair was obtained with the help of mathematical statistics methods.

On the basis of obtained load-elongation curves basic mechanical characteristics were determined: yield stress ($\sigma_T$, MPa), ultimate strength ($\sigma_\pi$, MPa), breaking elongation ($\varepsilon_p$, %) and initial tangent modulus of elasticity ($E_0$, GPa).

The values of initial tangent modulus of elasticity $E_0$ were determined by elongation curve derivation:

$$E_0 = \partial \sigma_T (\varepsilon_p) / \partial \varepsilon_p.$$

Regulatory document for performance of tests: GOST 20269-93 "Wool. Methods for Determination of Fiber Breaking Force".

Equipment: Instron 1122 universal breaking machine (Great Britain).

Test conditions: rate - 10 mm/min; temperature: 23±2 °C; sample base: 30 mm.

Number of tests: 20 (10 in each series)

The test results calculated with confidence coefficient of 95% are presented in Table 12.

Table 12. Results of tests

| Sample No. | Average diameter, mm | Yield stress, MPa | Ultimate strength, MPa | Breaking elongatio n, % | Initial tangent modulus of elasticity, GPa |
|---|---|---|---|---|---|
| 1. Treatment by Phases A, B and C | 0.053 | 206±14 | 316±23 | 57±6 | 7.5±0.7 |
| 2. Treatment by Phases A and B | 0.050 | 197±23 | 295±37 | 54±5 | 8.2±1 |

Example 15.

Experimentally it was established that when the polyisobutene to Versagel® ME 750 ratio was 2:1 (and higher percentage of polyisobutene) and the resulting formulation was too sticky and it was rather difficult to remove it from hair; when the polyisobutene to Versagel® ME 750 ratio was 1:2 (and higher percentage of Versagel® ME 750), the formulation became ineffective.

Example 16.

Experimentally it was established that when phases are in the ratio of 6:12:1, light hair is lightened very well (tone depth level (TDL) - 8), but for dark hair (TDL<7) it is not sufficient and excessive pigment in hair can give reddish or yellowish shade during bleaching.

INDUSTRIAL APPLICABILITY

In the result of performed tests it was established that the hair bleaching formulation of the invention does not have any cytotoxic, skin-irritant and sensitizing effect and can be used in cosmetic industry.

Claims

1. A formulation for hair bleaching that consists of three phases located separately from each other and mixed directly before use, where:

   - Phase A is a powder containing potassium persulfate, ammonium persulfate and sodium metasilicate;
   - Phase B is aqueous solution containing hydrogen peroxide in an acidic environment;

- Phase C is oil phase containing mineral oil, mixture of polyisobutene with mixture of hydrogenated polyisobutene with ethylene/propylene/styrene copolymer and butylene/ethylene/styrene copolymer, and conditioning agent is phenyl trimethicone, preservative agent is phenoxyethanol.

2. The hair bleaching formulation according to Claim 1, **characterized in that** its components in Phase C are in the following ratios, % by weight:

| | |
|---|---|
| polyisobutene | 10.0-35.0; |
| mixture of hydrogenated polyisobutene with ethylene/propylene/styrene copolymer and butylene/ethylene/styrene copolymer | 10.0-35.0; |
| phenyl trimethicone | 1.0-15; |
| phenoxyethanol | 0.1-1.0; |
| mineral oil | up to 100. |

3. The hair bleaching formulation according to any one of Claims 1 or 2, **characterized in that** Phase B additionally contains cetearyl alcohol, ceteareth-20, cetrimonium chloride, sodium stannate and phosphoric acid.

4. The hair bleaching formulation according to Claim 3, **characterized in that** its components in Phase B are in the following ratios, % by weight:

| | |
|---|---|
| hydrogen peroxide | 1.0-12.0; |
| cetearyl alcohol | 0.5-10.0; |
| ceteareth-20 | 0.1-8.0; |
| cetrimonium chloride | 0.1-5.0; |
| sodium stannate | 0.01-1.0; |
| phosphoric acid | 0.01-1.0; |
| water | up to 100. |

5. The hair bleaching formulation according to any one of Claims 1-4, **characterized in that** its components in Phase A are in the following ratios, % by weight:

| | |
|---|---|
| sodium metasilicate | 5.0-30.0; |
| ammonium persulfate | 10.0-40.0; |
| potassium persulfate | up to 100. |

6. The hair bleaching formulation according to any one of Claims 1-4, **characterized in that** Phase A additionally contains inorganic fillers, thickening agents, surfactant(s), mineral oil, complexones, perfume compositions and/or colorants, pigments.

7. The hair bleaching formulation according to Claim 6, **characterized in that** inorganic fillers in Phase A are selected from a group consisting of magnesium oxide, titanium dioxide, silica dioxide.

8. The hair bleaching formulation according to Claim 6, **characterized in that** the thickener in Phase A is selected from a group consisting of xanthan gum, guar hydroxypropyltrimonium chloride.

9. The hair bleaching formulation according to Claim 6, **characterized in that** surfactant in Phase A is selected from sodium lauryl sulfate.

10. The hair bleaching formulation according to Claim 6, **characterized in that** complexone in Phase A is selected from ethylenediaminetetraacetic acid tetrasodium.

11. The hair bleaching formulation according to Claim 6, **characterized in that** colorant in Phase A is selected from ultramarine.

12. The hair bleaching formulation according to Claim 1, **characterized in that**:

- Phase A contains, % by weight:

| | |
|---|---|
| sodium metasilicate | 5.0-30.0; |
| ammonium persulfate | 10.0-40.0; |
| potassium persulfate | up to 100; |

- Phase B contains, % by weight:

| | |
|---|---|
| hydrogen peroxide | 1.0-12.0; |
| water | up to 100; |

- Phase C contains, % by weight:

| | |
|---|---|
| polyisobutene | 10.0-35.0; |
| mixture of hydrogenated polyisobutene with ethylene/propylene/styrene copolymer and butylene/ethylene/styrene copolymer | 10.0-35.0; |
| mineral oil | up to 100. |

13. The hair bleaching formulation according to any one of Claims 1-12, **characterized in that** the formulation is intended for bleaching of light hair and that Phase A, Phase B and Phase C are in the ratio of 6:12:1.

**Patentansprüche**

1. Formulierung zum Bleichen von Haaren, die aus drei voneinander getrennten Phasen besteht, die unmittelbar vor der Anwendung gemischt werden, wobei:

- Phase A ist ein Pulver, das Kaliumpersulfat, Ammoniumpersulfat und Natriummetasilikat enthält;
- Phase B ist eine wässrige Lösung, die Wasserstoffperoxid in einer sauren Umgebung enthält;
- Phase C ist eine Ölphase, die Mineralöl, eine Mischung aus Polyisobuten mit einer Mischung aus hydriertem Polyisobuten mit Ethylen/Propylen/Styrol-Copolymer und Butylen/Ethylen/Styrol-Copolymer enthält, und das Konditionierungsmittel ist Phenyltrimethicon, das Konservierungsmittel ist Phenoxyethanol.

2. Die Haarbleichmittelformulierung gemäß Anspruch 1, **dadurch gekennzeichnet, dass** die Bestandteile in Phase C in den folgenden Verhältnissen, in Gewichtsprozent, vorliegen

| | |
|---|---|
| Polyisobuten | 10,0-35,0; |
| Mischung von hydriertem Polyisobuten mit Ethylen/Propylen/Styrol-Copolymer und Butylen/Ethylen/Styrol-Copolymer | 10,0-35,0; |
| Phenyltrimethicon | 1,0-15; |
| Phenoxyethanol | 0,1-1,0; |
| Mineralöl | bis zu 100. |

3. Die Haarbleichformulierung gemäß einem der Ansprüche 1 oder 2, **dadurch gekennzeichnet, dass** Phase B zusätzlich Cetearylalkohol, Ceteareth-20, Cetrimoniumchlorid, Natriumstannat und Phosphorsäure enthält.

4. Die Haarbleichmittelformulierung gemäß Anspruch 3, **dadurch gekennzeichnet, dass** die Bestandteile in Phase B in den folgenden Verhältnissen, in Gewichtsprozent, vorliegen:

| | |
|---|---|
| Wasserstoffperoxyd | 1,0-12,0; |
| Cetearylalkohol | 0,5-10,0; |
| Ceteareth-20 | 0,1-8,0; |
| Cetrimoniumchlorid | 0,1-5,0; |
| Natriumstannat | 0,01-1,0; |
| Phosphorsäure | 0,01-1,0; |
| Wasser | bis zu 100 |

5.  Die Haarbleichformulierung gemäß einem der Ansprüche 1 bis 4, **dadurch gekennzeichnet, dass** die Bestandteile in Phase A in den folgenden Verhältnissen, in Gewichtsprozent, vorliegen

| | |
|---|---|
| Natriummetasilikat | 5,0-30,0; |
| Ammoniumpersulfat | 10,0-40,0; |
| Kaliumpersulfat | bis zu 100 |

6.  Die Haarbleichformulierung gemäß einem der Ansprüche 1 bis 4, **dadurch gekennzeichnet, dass** Phase A zusätzlich anorganische Füllstoffe, Verdickungsmittel, Tensid(e), Mineralöl, Komplexone, Parfümkompositionen und/oder Farbstoffe, Pigmente enthält.

7.  Die Haarbleichende Formulierung gemäß Anspruch 6, **dadurch gekennzeichnet, dass** die anorganischen Füllstoffe in Phase A ausgewählt sind aus einer Gruppe bestehend aus Magnesiumoxid, Titandioxid und Siliciumdioxid.

8.  Die Haarbleichmittelformulierung gemäß Anspruch 6, **dadurch gekennzeichnet, dass** das Verdickungsmittel in Phase A ausgewählt ist aus einer Gruppe bestehend aus Xanthangummi, Guarhydroxypropyltrimoniumchlorid.

9.  Die Haarbleichmittelformulierung gemäß Anspruch 6, **dadurch gekennzeichnet, dass** das Tensid in Phase A ausgewählt ist aus Natriumlaurylsulfat.

10. Die Haarbleichmittelformulierung gemäß Anspruch 6, **dadurch gekennzeichnet, dass** das Komplexen in Phase A ausgewählt ist aus Ethylendiamintetraessigsäure-Tetranatrium.

11. Die Haarbleichmittelformulierung gemäß Anspruch 6, **dadurch gekennzeichnet, dass** der Farbstoff in Phase A aus Ultramarin ausgewählt ist.

12. Die Haarbleichmittelformulierung gemäß Anspruch 1, **dadurch gekennzeichnet, dass**:

    - Phase A enthält, in Gewichtsprozent:

| | |
|---|---|
| Natriummetasilikat | 5,0-30,0; |
| Ammoniumpersulfat | 10,0-40,0; |
| Kaliumpersulfat | bis zu 100; |

    - Phase B enthält, in Gewichtsprozent:

| | |
|---|---|
| Wasserstoffperoxyd | 1,0-12,0; |
| Wasser | bis zu 100; |

    - Phase C enthält, in Gewichtsprozent:

| | |
|---|---|
| Polyisobuten | 10,0-35,0; |

| | |
|---|---|
| Mischung von hydriertem Polyisobuten mit Ethylen/Propylen/Styrol-Copolymer und Butylen/Ethylen/ Styrol-Copolymer | 10,0-35,0; |
| Mineralöl | bis zu 100. |

**13.** Die Haarbleichmittelformulierung gemäß einem der Ansprüche 1 bis 12, **dadurch gekennzeichnet, dass** die Formulierung zum Bleichen von hellem Haar bestimmt ist und dass Phase A, Phase B und Phase C im Verhältnis 6:12:1 vorliegen.

**Revendications**

**1.** Une formulation pour la décoloration des cheveux qui consiste en trois phases situées séparément les unes des autres et mélangées directement avant l'utilisation, où

- la phase A est une poudre contenant du persulfate de potassium, du persulfate d'ammonium et du métasilicate de sodium ;
- la phase B est une solution aqueuse contenant du peroxyde d'hydrogène dans un environnement acide ;
- la phase C est une phase huileuse contenant de l'huile minérale, un mélange de polyisobutène avec un mélange de polyisobutène hydrogéné avec un copolymère éthylène/propylène/styrène et un copolymère butylène/éthylène/styrène, et l'agent de conditionnement est la triméthicone de phényle, l'agent de conservation est le phénoxyéthanol.

**2.** La formulation de décoloration des cheveux selon la revendication 1, **caractérisée en ce que** ses composants dans la phase C sont dans les rapports suivants, en % en poids :

| | |
|---|---|
| polyisobutène : | 10,0-35,0 ; |
| mélange de polyisobutène hydrogéné avec duéthylène/propylène/styrène et du butylène/ éthylène/styrène : copolymère avec du | 10,0-35,0 ; |
| triméthicone de phényle : | 1,0-15 ; |
| phénoxyéthanol : | 0,1-1,0 ; |
| huile minérale : | jusqu'à 100. |

**3.** La formulation de décoloration des cheveux selon l'une quelconque des revendications 1 ou 2, **caractérisée en ce que** la phase B contient en outre de l'alcool cétéarylique, du cétéareth-20, du chlorure de cétrimonium, du stannate de sodium et de l'acide phosphorique.

**4.** La formulation de décoloration des cheveux selon la revendication 3, **caractérisée en ce que** ses composants dans la phase B sont dans les rapports suivants, en % en poids :

| | |
|---|---|
| peroxyde d'hydrogène : | 1,0-12,0 ; |
| alcool cétéarylique : | 0,5-10,0 ; |
| cétéareth-20 : | 0,1-8,0 ; |
| chlorure de cétrimonium : | 0,1-5,0 ; |
| stannate de sodium : | 0,01-1,0 ; |
| acide phosphorique : | 0,01-1,0 ; |
| eau : | jusqu'à 100. |

**5.** La formulation de décoloration des cheveux selon l'une quelconque des revendications 1 à 4, **caractérisée en ce que** ses composants dans la phase A sont dans les rapports suivants, en % en poids :

| | |
|---|---|
| métasilicate de sodium : | 5,0-30,0 |

(suite)

persulfate d'ammonium :     10,0-40,0
persulfate de potassium :   jusqu'à 100.

6.  La formulation de décoloration des cheveux selon l'une quelconque des revendications 1 à 4, **caractérisée en ce que** la phase A contient en outre des charges inorganiques, des agents épaississants, des surfactants, de l'huile minérale, des complexones, des compositions parfumées et/ou des colorants, des pigments.

7.  La formulation de décoloration des cheveux selon la revendication 6, **caractérisée en ce que** des charges inorganiques dans la phase A sont choisies dans un groupe constitué par l'oxyde de magnésium, le dioxyde de titane, le dioxyde de silice.

8.  La formulation de décoloration des cheveux selon la revendication 6, **caractérisée en ce que** l'épaississant dans la phase A est choisi dans un groupe constitué par la gomme xanthane, le chlorure de guar hydroxypropyltrimonium.

9.  La formulation de décoloration des cheveux selon la revendication 6, **caractérisée en ce que** le tensioactif dans la phase A est choisi parmi le lauryl sulfate de sodium.

10. La formulation de décoloration des cheveux selon la revendication 6, **caractérisée en ce que** la complexone dans la phase A est choisie parmi l'acide éthylènediaminetétraacétique tétrasodique.

11. La formulation de décoloration des cheveux selon la revendication 6, **caractérisée en ce que** le colorant dans la phase A est choisi parmi l'outremer.

12. La formulation de décoloration des cheveux selon la revendication 1, **caractérisée en ce que** :

    - la phase A contient, en % en poids :

        métasilicate de sodium :    5,0-30,0 ;
        persulfate d'ammonium :     10,0-40,0 ;
        persulfate de potassium :   jusqu'à 100 ;

    - la phase B contient, en % en poids :

        peroxyde d'hydrogène :   1,0-12,0 ;
        eau :                    jusqu'à 100 ;

    - la phase C contient, en % en poids :

polyisobutène :                                                                                      10,0-35,0 ;
mélange de polyisobutène hydrogéné avec du copolymère éthylène/propylène/styrène et du       10,0-35,0
copolymère butylène/éthylène/styrène :
huile minérale :                                                                                     jusqu'à 100.

13. La formulation de décoloration des cheveux selon l'une quelconque des revendications 1 à 12, **caractérisée en ce que** la formulation est destinée à la décoloration des cheveux clairs et que la phase A, la phase B et la phase C sont dans le rapport 6 : 12 : 1.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2017128334 A1
- DE 102006053343 A1
- DE 102006022214 A1
- EP 1430873 A1
- US 6273920 B1
- FR 2870736 B1
- GB 1059986 A

- WO 2011121010 A1
- US 3649159 A
- DE 102004030178 A1
- DE 102005019420 A1
- US 5989530 A
- US 20070169285 A1
- WO 2009134875 A2